(19) Europäisches Patentamt / European Patent Office / Office européen des brevets

(11) **EP 3 784 136 B1**

(12) **EUROPEAN PATENT SPECIFICATION**

(45) Date of publication and mention
of the grant of the patent:
**26.06.2024 Bulletin 2024/26**

(21) Application number: **19720834.1**

(22) Date of filing: **26.04.2019**

(51) International Patent Classification (IPC):
*A61B 6/00* *(2024.01)*      *A61B 17/32* *(2006.01)*
*F16F 7/10* *(2006.01)*      *A61B 8/14* *(2006.01)*
*A61B 8/00* *(2006.01)*      *A61B 8/08* *(2006.01)*
*B06B 1/12* *(2006.01)*      *B06B 1/18* *(2006.01)*
*G01S 15/89* *(2006.01)*     *G10K 11/35* *(2006.01)*
*A61C 9/00* *(2006.01)*      *F16F 15/14* *(2006.01)*

(52) Cooperative Patent Classification (CPC):
**A61B 8/4461; A61B 8/14; A61B 8/483;**
**A61C 9/0086; F16F 7/1005; F16F 7/1028;**
**G01S 15/8945; G10K 11/352;** A61B 8/4254

(86) International application number:
**PCT/EP2019/060781**

(87) International publication number:
**WO 2019/207126 (31.10.2019 Gazette 2019/44)**

(54) **DRIVE UNIT WITH OSCILLATING ELEMENT**

ANTRIEBSEINHEIT MIT SCHWINGENDEM ELEMENT

UNITÉ D'ENTRAÎNEMENT POURVUE D'UN ÉLÉMENT OSCILLANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.04.2018 DE 102018110274**
**27.04.2018 DE 102018110273**

(43) Date of publication of application:
**03.03.2021 Bulletin 2021/09**

(73) Proprietor: **Dentacon GmbH**
**66978 Donsieders (DE)**

(72) Inventors:
• **HESSE, Steffen**
**98693 Ilmenau (DE)**
• **KATZSCHMANN, Michael**
**98693 Ilmenau (DE)**
• **MOHR, Hans-Ulrich**
**98701 Großbreitenbach (DE)**
• **SCHÄFFEL, Christoph**
**98693 Ilmenau (DE)**

(74) Representative: **Sonnenberg Harrison
Partnerschaft mbB et al
Herzogspitalstraße 10a
80331 München (DE)**

(56) References cited:
EP-A1- 1 737 110      DE-A1-102012 111 958
DE-B4- 10 019 226      US-A1- 2012 095 472

## Description

### Reference to cross-related applications

This application claims the priority of DE 10 2018 110 274.7, filed on 27 April 2018, and of DE 10 2018 110 273.9, filed on 27 April 2018.

### Background of the invention

The invention relates to a drive unit having an oscillating element. The drive unit has an oscillator for generating an oscillating movement, and a compensator for generating a compensating movement for compensating the vibrations occurring at the drive unit.

A preferred field of application of such a drive unit is the oscillating movement of an oscillating element, such as, for example, a laser light source or a sensor, when in the particular application an oscillating movement of the oscillating element is desired.

One example of a preferred field of application is ultrasonic scanning, in which the oscillating element is an ultrasonic sensor. The oscillating movement of the ultrasonic sensor can inter alia be used to record, based on ultrasound, body parts arranged in cavities, in particular shapes of teeth and jaws of a patient.

EP 0 035 307 A2 discloses a dental tomography device with a carrier, which is rotatable about a vertical axis, and to which a radiation source and a film holder aligned thereto are attached. Such devices are employed to generate panoramic recordings of the dental arch.

Ultrasonic scanners are widely employed for diagnostic purposes and are an instrument of choice due to high biocompatibility based on the absence of radiation exposure. The analysis of images generated by ultrasound generally requires extensive experience due to the possible presence of artefacts in the generated images. Furthermore, the handling of mobile scanning devices requires particular skills, involves intense computation, and/or results in reduced quality of the image, due to vibrations and/or wobbling, possibly generated in the device or from external sources.

An electromechanical linear drive with torque compensation is known from DE 100 19 226 B4. The linear drive has a first actuator, movable along a rectilinear first movement path in both directions, and a driven first load, rigidly connected thereto. Furthermore, an electromechanical torque compensation drive is provided, which comprises a second actuator, having a second movement path parallel to the first movement path, and a driven second load, rigidly connected to the second actuator. A compensation control device provides a control, synchronized with the first actuator, of the second actuator to move in phase opposition relative to the first actuator. The second load comprises a considerably greater mass than the first load. The first movement path has a greater amplitude than the second movement path. Such a drive can provide a reduction in vibrations with stationary devices but is not suitable for application in mobile devices that during use undergo constant position changes.

An object of the present invention is to provide a drive unit for generating a fast oscillating movement. Such a drive unit is of a compact design and to be employed in, for instance, mobile devices. In particular, the drive unit is to be configured to be a component of a precisely guidable hand-held device.

This object is basically solved by a device having the features of claim 1. Preferred embodiments are defined in the dependent claims, hereinbelow, and in the description of the preferred embodiments.

In a particularly preferred embodiment, the drive unit can serve as an ultrasonic scanner for generating and recording ultrasound, in particular in diagnostic imaging. Such an ultrasonic scanner is of a compact design and suited for use in the oral cavity of a patient for a recording of the shape of the teeth and the jaws. In particular, the ultrasonic scanner is to be configured to be a component of a hand-held device, such that during use no further stands, mounts, or positioning devices are required.

The drive unit according to an embodiment of the invention comprises an oscillator that is excited by a first actuator to move along a longitudinal axis. The oscillator may be moved with a pre-determined frequency and a first amplitude, to generate an oscillating movement. Furthermore, a compensator is provided that is excited by a second actuator along, or parallel to, the longitudinal axis to move in phase opposition. The compensator may be moved with the pre-determined frequency and a second amplitude, to generate a compensating movement. Preferably, the oscillator and the compensator are arranged in an aligned or flush manner.

An oscillating or compensating movement is to be understood to be an alternating movement in the direction of, or parallel to, the longitudinal axis at an appropriate frequency. The oscillating movement is preferably used for the movement of an oscillating element (further described below), which may be, for example, a laser diode, an ultrasonic probe or any kind of sensor that are to be moved with a small stroke.

In a further aspect, the drive unit further comprises a guide unit that guides, in an axially movable manner, the oscillator in a first guide and the compensator in a second guide. The two guides preferably extend in the direction of, or parallel to, the longitudinal axis and allow guiding the movable parts with minimal friction. The first guide is a first air guide, and the second guide is a second air guide.

In yet a further aspect of the invention, a further component of the drive unit is a position sensor that provides a positioning signal relating to the position of the longitudinal axis. The positioning signal may be representative of the deviation of the longitudinal axis from the horizontal. The positioning signal correlates therefore with the proportion of the weight or inertial forces of the oscillator and the compensator, which is to be provided by the corresponding actuator as the steady component of the actuator force in order to hold the zero position in place. This component is equal to zero, when the oscillator and compensator are exactly balanced.

The drive unit comprises according to a preferred embodiment an oscillating element, which, for example, generates and emits electromagnetic waves or acoustic waves, and/or which receives, and converts into an electrical response signal, radiation or waves from an external source. The response signal may be further processed by downstream units to give, for example, an image of a scanned or sampled object.

Finally, the drive unit comprises a control unit that controls the first and second actuators to drive the oscillator and the compensator to move with the same frequency but in phase opposition along the longitudinal axis or on parallel axes. The control unit provides the driving power for the oscillator and the compensator. The driving power may be provided in consideration of the positioning signal. The driving power may thus be provided such that, independent of the spatial position of longitudinal axis, the compensator momentums, generated by the movement of the compensator, are equal in magnitude to the oscillator momentums, generated by the movement of the oscillator. Due to the phase offset of 180°, the momentums or the forces generating undesired vibrations cancel each other. This enables the oscillator to carry out an exact, precisely controlled oscillating movement, which is of advantage for precise movement of the oscillating element.

Preferably, the drive unit comprises at least one translation sensor that senses the oscillating movement of the oscillator and provides a translation signal to the control unit. According to a further modified embodiment, a second translation sensor is provided that senses the compensating movement of the compensator and provides a corresponding signal to the control unit.

According to a preferred embodiment, the oscillator comprises an oscillatory carriage. The oscillatory carriage may be guided in the first guide along the longitudinal axis in an axially movable manner. The oscillatory carriage has a, for example, prism-shaped cross-section.

The first guide is continually supplied with pressurized air, to establish the required air cushion. A guiding area is divided into a proximal supporting area and a distal sealing area. Between the supporting area and the sealing area, a blow-out opening is provided, from which a certain proportion of the pressurized air is continually blown out, by means of which, where applicable, fluid entering via the sealing area is blown out and cannot enter the supporting area of the air guide.

It is particularly advantageous if the distal end of the oscillatory carriage axially extends beyond the sealing area into a fluid area, into which a supply pipe for supplying fluid leads. The fluid area preferably has an opening, from which fluid can exit. The fluid can, for instance, be water for purposes of cooling.

The oscillating element is preferably attached to the distal end of the oscillatory carriage, the distal end being located in the fluid area, such that the oscillating element is arranged in the opening.

In an advantageous embodiment of the drive unit, the first and second actuators are each formed by a plunger coil drive. Other drives having corresponding dynamics are also possible.

Preferably, the oscillator and compensator have an equal moved mass. In modified embodiments, the masses can be chosen to be different, with the momentums generated by the movement being adaptable to one another by altering acceleration values.

In a preferred embodiment, the position sensor may be an inclination sensor or an acceleration sensor. Alternatively, the position may be indirectly determined by means of one or more inertial sensors. Likewise, a combination of several sensors is possible.

According to a particularly preferred embodiment, the drive unit comprises an elongated housing, which can be gripped, or taken hold of, in a middle portion by a user. The housing be formed to by cylindrical in portions, and can have in the gripping area an, in haptic terms, pleasant surface. At a front end, the housing preferably has the opening, and at a rear end supply ports for pressurized air and fluid are provided.

According to an embodiment, by employing the position sensor that detects the deviation of the longitudinal axis from the horizontal, the actual inclination of the drive unit can be determined and taken into account when defining the control function of the compensator. Moreover, the positioning signal provided by the position sensor can also be used for further purposes.

Furthermore, asymmetries of distribution of mass between the oscillator and the compensator can be controlled, for instance by evaluating a further signal from the acceleration sensor. It is likewise possible to detect, and control, asymmetries of the frictional conditions and of further interfering forces (e.g. caused by moved electrical wiring).

In one aspect, the oscillating element is an ultrasonic sensor. The drive unit comprising the ultrasonic sensor forms an ultrasonic scanner.

The oscillating movement, as understood in this disclosure, of the ultrasonic sensor allows to move the ultrasonic sensor with a small stroke during a scanning operation, such that even fine contours of a body to be scanned, such as a tooth, can be registered in detail. The ultrasonic sensor generates and emits ultrasound, and receives, and converts into an electrical response signal, response waves reflected from the body, for example a tooth, exposed to said ultrasound. The downstream units further process the response signal to give an image of the scanned body, e.g. a tooth. The providing by the control unit of the driving power for the oscillator and the compensator, e.g. at a phase offset of 180°, enables an exact, precisely controlled oscillating movement, which is necessary for a satisfactory result of the scanning operation.

In this aspect, the fluid is a coupling medium, preferably water. The fluid area is a coupling area, and the opening is a coupling opening, from which the coupling medium and ultrasound may exit. The coupling medium is required to minimize losses in transmitting both the ultrasonic waves to the body to be scanned, such as a tooth, and the response waves back to the ultrasonic sensor, as a gap-free abutment of the oscillating ultrasonic sensor against the body is impossible.

The ultrasonic sensor is preferably attached to the distal end of the oscillatory carriage, the distal end being located in the coupling area, such that the ultrasonic sensor is arranged in the coupling opening.

The housing of the ultrasonic scanner may have the coupling opening at a front end and may have the supply ports for pressurized air and coupling medium, such as water, at the rear end.

The positioning signal provided by the position sensor may be used for generating a three-dimensional image, which is represented by the response waves, of e.g. the body, such as a tooth.

Further advantages and details of the invention will become clear from the following description of preferred embodiments with reference to the drawings.

**Brief description of the drawings**

Fig. 1 is a perspective view of an aspect of drive unit according to the invention;

Fig. 2 shows components of the drive unit in a top view and in a lateral sectional view; and

Fig. 3 is a detailed sectional view of a first air guide with an oscillator.

**Detailed description of the embodiments**

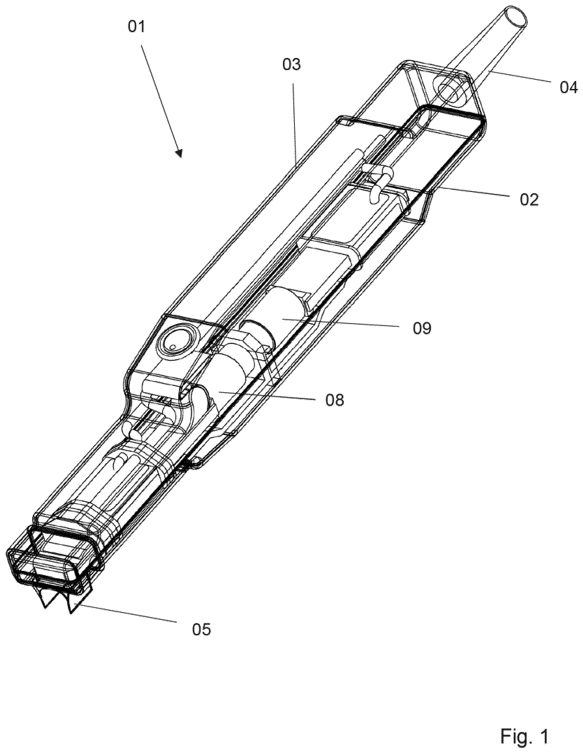

Fig. 1 shows, in a perspective view, one aspect of the invention. The drive unit 01 may have a housing 02, of which solely the contours are drawn for rendering visible some of parts located within the housing. The housing 02 may, for example, be made of plastic and has a grip portion 03 that is gripped or taken hold of, by the user during use. At a rear end of the housing 02, supply ports 04 are arranged, via which pressurized air, water, and electric power can be supplied. Furthermore, data transport can be carried out via the supply ports, to connect the drive unit to external data processing devices.

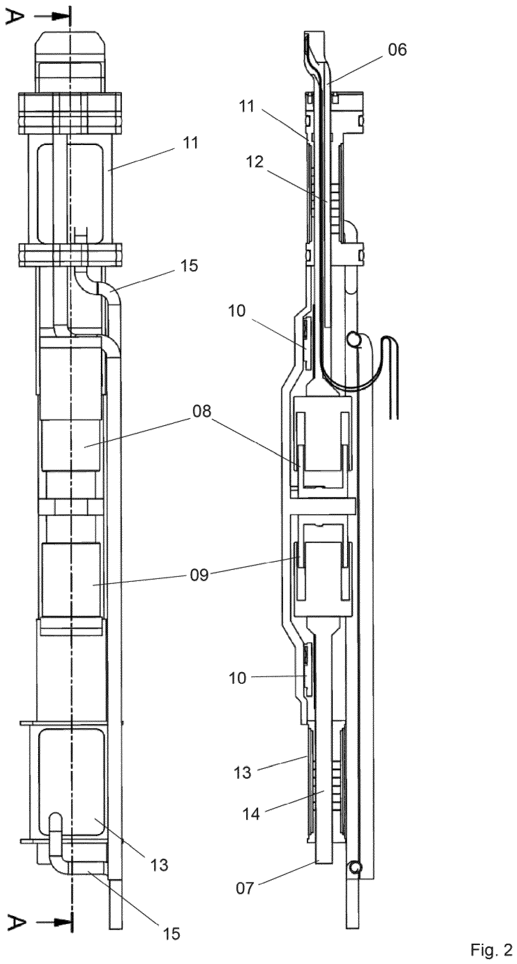

Fig. 2 shows several components of the drive unit 01, in a top view and in a lateral view along a sectional line A-A. In case the drive unit has a housing 02, the components may be located within the housing 02. An oscillator 06 and a compensator 07 are arranged in an axially aligned or flush manner and are displaceably guided in the direction of a common longitudinal axis. The oscillator 06 is excited by a first actuator 08 to move in an oscillating manner in the direction of the longitudinal axis. The compensator is excited in an equal manner by a second actuator 09 to move in a compensating manner. The first and second actuator 08 and 09 are configured to be plunger coil drives or oscillatory coil drives. The plunger coil drives are controlled by a control unit (not shown) in phase opposition, which minimizes undesired oscillations at the drive unit. The frequency, at which the oscillator 06 and the compensator 07 are driven to move, is for example 20 Hz, and the amplitude of the two movements is for example ± 2 mm.

A guide unit comprises a first air guide 11, in which an oscillatory carriage 12 of the oscillator 06 is guided in an axially movable manner, and a second air guide 13, in which a compensatory carriage 14 of the compensator 07 is guided in an axially movable manner. The two air guides 11, 13 are supplied with pressurized air via a pressurized air pipe 15. This enables aerostatic guiding of the oscillator and the compensator to minimize the friction, the wear, the speed fluctuations, the noise level as well as maintenance requirements. This results in a high reliability and durability of the entire drive unit due to the contactless and frictionless operation of all mechanically moved elements.

The oscillatory carriage 12 and the compensatory carriage 14 are arranged in an aligned or flush and axially opposing manner. The plunger coil drives 08, 09 each move one of the said carriages. To avoid moved electrical wiring, the coils of the actuators are attached to a frame element in a symmetrically opposing and aligned manner. The iron core of each plunger coil drive is connected with one of the two carriages 12, 14 in a fixed and aligned manner.

The movement of at least one of the two carriages 12, 14 is sensed by a translation sensor 10 and is provided to the control unit as a control parameter. To minimize undesired vibrations of the frame of the drive unit, both plunger coil drives are controlled in exact phase opposition. Preferably, the compensatory carriage 14 has a mass exactly equal to the mass of the oscillatory carriage 12. Preferably, both plunger coil drives 08, 09 are arranged in a control loop, wherein the position of each carriage 12, 14 relative to the frame is sensed in a precise and contactless manner by, for example, an optical translation sensor 10, and the control defines a positional curve in phase opposition and of equal amplitude, for example a sine and an inverted sine, for both carriages.

For any spatial position of the movement axes of the two plunger coil drives 08, 09, the vibrational excitation of the frame can be controlled to be exactly zero. Depending on the inclination of the longitudinal axis (movement axis), in addition to the imposed movement function, a constant force has to be provided by the plunger coil drives for supporting the weight of the carriage,

$$Fg = m\, g\, \sin\varphi,$$

with m = mass of the carriage, and

$\varphi$ = inclination angle relative to the horizontal.

The inclination of the drive unit, i.e. of the longitudinal axis relative to the horizontal, is preferably sensed by means of an inclination sensor, and is used for feedforward control of the controllers of the carriage movement. The efficiency of the compensation of vibrations is substantially dependent on how identical the movement behaviors of the two symmetrically opposed carriages 12, 14 are. The use of the air guide 11, 13 is therefore advantageous for the achievable degree of compensation of vibrations.

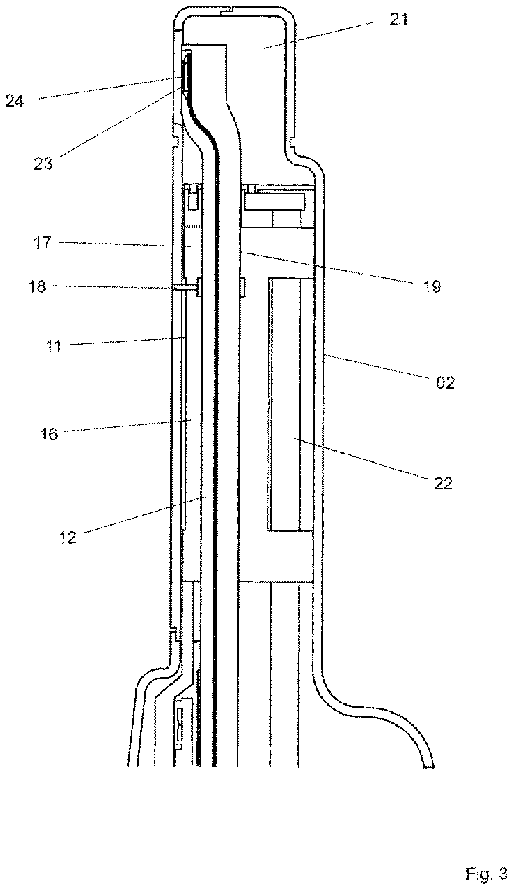

Fig. 3 shows a detailed sectional view of the first air guide 11 with the oscillatory carriage 12 guided therein. The first air guide 11 comprises a proximal supporting area 16, a distal sealing area 17, and a blow-out opening 18 arranged between the supporting area 16 and the sealing area 17. In the sealing area 17, a gap seal of for example 5 $\mu$m remains between the axially movable oscillatory carriage 12 and the guide surface, allowing frictionless sliding of the oscillatory carriage 12, but having a sealing effect with respect to liquids. Small amounts of liquid, which nevertheless are able to enter through the gap sealing 19 from a fluid area 21 filled with a fluid, are transported by the pressurized air to the surroundings via the blow-out opening 18, such that they do not entre the supporting area 16. In the supporting area 16, there is also a gap to form the air guide.

The fluid area 21 is supplied with a fluid, e.g. water, via a supply pipe 22. The fluid can exit from the fluid area 21 at an opening 23 for, e.g., purposes of cooling.

In the shown embodiment, an oscillating element 24 is arranged at the distal end of the oscillatory carriage 12 that is located in the fluid area 21. The oscillating element 24, e.g. a laser light source to be moved or a sensor, is located in the opening 23, such that accessing the oscillating element is possible. The usage of the air guide enables wet / dry media separation. Although the oscillating element 24 operates in fluidic surroundings, an entering of fluid into the main part of the drive unit can be reliably avoided.

According to a further aspect of drive unit according to the invention, the oscillating element 24 may be an ultrasonic sensor 24. The drive unit 01 comprising the ultrasonic sensor 24 forms an ultrasonic scanner 01.

In this aspect, the fluid area 21 may be a coupling area 21 that is filled with coupling medium. The drive unit may comprise the housing 02, and at the front end of the housing 02, elastic enclosing lips 05 may be arranged that enclose the object to be scanned, e.g. a body such as a tooth of a patient, for reducing lateral draining of water supplied as a coupling medium.

In this aspect, the coupling area 21 is supplied with a coupling medium, preferably water, via a supply pipe 22. The coupling medium can exit from the coupling area 21 at a coupling opening 23 in order to be led to the tooth to be scanned.

The ultrasonic sensor 24 is arranged at the distal end of the oscillatory carriage 12 that is located in the coupling area 21. The ultrasonic sensor 24 is located in the window of the coupling opening 23, such that ultrasound can exit, and reflected response waves can enter. The usage of the air guide enables wet / dry media separation. Although the ultrasonic sensor 24 operates in fluidic surroundings, entering of coupling medium into the main part of the drive unit can be reliably avoided.

**Reference numerals**

| 01 | Drive unit / Ultrasonic scanner |
|----|----|
| 02 | Housing |
| 03 | Grip portion |
| 04 | Supply ports |
| 05 | Enclosing lips |
| 06 | Oscillator |
| 07 | Compensator |
| 08 | First actuator |
| 09 | Second actuator |
| 10 | Translation sensor |
| 11 | First air guide |
| 12 | Oscillatory carriage |
| 13 | Second air guide |
| 14 | Compensator carriage |
| 15 | Pressurized air pipe |
| 16 | Support area |
| 17 | Sealing area |
| 18 | Blow-out opening |
| 19 | Gap sealing |
| 21 | Fluid area / Coupling area |
| 22 | Supply pipe |
| 23 | Opening / Coupling opening |
| 24 | Oscillating element / Ultrasonic sensor |

**Claims**

**1.** A drive unit (01) for diagnostic imaging, comprising:

- an oscillator (06) that is drivable by a first actuator (08) to move along a longitudinal axis to generate an oscillating movement;

- a compensator (07) that is drivable by a second actuator (09) along, or parallel to, the longitudinal axis to move in phase opposition to generate a compensating movement;

- a guide unit that guides, in an axially movable manner, the oscillator (06) in a first guide (11) and the compensator (07) in a second guide (13);

- a control unit that controls the first and second actuators (08, 09) to drive the oscillator (06) and the compensator (07),

wherein the oscillator (06) comprises an oscillating element (24) that is a laser light source or an ultrasonic sensor, and

the first guide (11) is a first air guide (11), and the second guide (13) is a second air guide (13).

2. The drive unit (01) of claim 1, further comprising a position sensor that provides a positioning signal relating to the position of the longitudinal axis, preferably the position sensor is an inclination sensor or an inertial sensor, or a combination of such sensors.

3. The drive unit (01) of claim 2, wherein the control unit controls the oscillator and the compensator (07) in consideration of the position of the longitudinal axis.

4. The drive unit (01) of one of claims 1 to 3, further comprising a translation sensor (10) that senses the oscillating movement and the compensating movement and provides a translation signal to the control unit.

5. The drive unit (01) of one of claims 1 to 4, wherein the oscillator (06) comprises an oscillatory carriage (12) that is guided in the first guide (11).

6. The drive unit (01) of any one of claims 1 to 5, wherein the oscillating element is attached to a distal end of the oscillatory carriage (12).

7. The drive unit (01) of one of claims 2 to 6, wherein the first air guide (11) comprises a supply of pressurized air, a proximal supporting area (16), a distal sealing area (17), and a blow-out opening arranged between the supporting area (16) and the sealing area (17).

8. The drive unit (10) of claim 7, wherein the distal end of the oscillatory carriage (12) axially extends beyond the sealing area (17) into a fluid area (21), into which a supply pipe (22) leads for supplying fluid, the fluid area (21) comprising an opening (23) for the fluid to exit from.

9. The drive unit (01) of claim 8, wherein the fluid is a coupling fluid, and the fluid area is a coupling area.

10. The drive unit (01) of claim 8 or 9, wherein the opening (23) is a coupling opening (23) for coupling medium and/or ultrasound to exit from.

11. The drive unit (01) of one of claims 8 to 10, wherein enclosing lips (05) are arranged at the opening (23) that guide the fluid.

12. The drive unit (01) of one of claims 1 to 11, wherein the first and second actuators (08, 09) are each formed by a plunger coil drive.

13. The drive unit (01) of one of claims 1 to 12, wherein the oscillator (06) and the compensator (07) have an equal moved mass.

14. The drive unit (01) of one of claims 1 to 13, further comprising an elongated housing, which can be gripped by a user, and which has supply ports, preferably for pressurized air and fluid.

**Patentansprüche**

1. Antriebseinheit (01) für diagnostische Bildgebung, umfassend:

einen Oszillator (06), der durch einen ersten Aktuator (08) entlang einer Längsachse antreibbar ist, um eine oszillierende Bewegung zu erzeugen;

einen Kompensator (07), der durch einen zweiten Aktuator entlang oder parallel zur Längsachse antreibbar ist, um in Gegenphase eine kompensierende Bewegung zu erzeugen;

eine Führungseinheit, die den Oszillator (06) in einer ersten Führung (11) und den Kompensator (07) in einer zweiten Führung (13) axial beweglich führt;

eine Steuereinheit, die die ersten und zweiten Aktuatoren (08, 09) steuert, um den Oszillator (06) und den Kompensator (07) anzutreiben,

wobei der Oszillator (06) ein oszillierendes Element (24) umfasst, das eine Laserlichtquelle oder ein Ultraschallsensor ist, und

die erste Führung (11) eine erste Luftführung (11) ist und die zweite Führung (13) eine zweite Luftführung (13).

2. Antriebseinheit (01) nach Anspruch 1, weiter umfassend einen Positionssensor, der ein Positionssignal bezüglich der Position der Längsachse liefert, vorzugsweise ist der Positionssensor ein Neigungssensor oder ein Inertialsensor oder eine Kombination solcher Sensoren.

**3.** Antriebseinheit (01) nach Anspruch 2, wobei die Steuereinheit den Oszillator und den Kompensator (07) unter Berücksichtigung der Position der Längsachse steuert.

**4.** Antriebseinheit (01) nach einem der Ansprüche 1 bis 3, weiter umfassend einen Translationssensor (10), der die oszillierende Bewegung und die kompensierende Bewegung erfasst und ein Translationssignal an die Steuereinheit liefert.

**5.** Antriebseinheit (01) nach einem der Ansprüche 1 bis 4, wobei der Oszillator (06) einen oszillierenden Schlitten (12) umfasst, der in der ersten Führung (11) geführt wird.

**6.** Antriebseinheit (01) nach einem der Ansprüche 1 bis 5, wobei das oszillierende Element am distalen Ende des oszillierenden Schlittens (12) angebracht ist.

**7.** Antriebseinheit (01) nach einem der Ansprüche 2 bis 6, wobei die erste Luftführung (11) eine Versorgung mit Druckluft, einen proximalen Stützbereich (16), einen distalen Dichtungsbereich (17) und eine Ausblasöffnung umfasst, die zwischen dem Stützbereich (16) und dem Dichtungsbereich (17) angeordnet ist.

**8.** Antriebseinheit (10) nach Anspruch 7, wobei das distale Ende des oszillierenden Schlittens (12) axial über den Dichtungsbereich (17) in einen Fluidbereich (21) hinausragt, in den eine Versorgungsleitung (22) für die Zufuhr von Fluid mündet, wobei der Fluidbereich (21) eine Öffnung (23) zum Austritt des Fluids aufweist.

**9.** Antriebseinheit (01) nach Anspruch 8, wobei das Fluid ein Kopplungsfluid ist und der Fluidbereich ein Kopplungsbereich.

**10.** Antriebseinheit (01) nach Anspruch 8 oder 9, wobei die Öffnung (23) eine Kopplungsöffnung (23) zum Austritt des Kopplungsmediums und/oder Ultraschall ist.

**11.** Antriebseinheit (01) nach einem der Ansprüche 8 bis 10, wobei an der Öffnung (23) umschließende Lippen (05) angeordnet sind, die das Fluid leiten.

**12.** Antriebseinheit (01) nach einem der Ansprüche 1 bis 11, wobei die ersten und zweiten Aktuatoren (08, 09) jeweils durch einen Kolbenspulantrieb gebildet sind.

**13.** Antriebseinheit (01) nach einem der Ansprüche 1 bis 12, wobei der Oszillator (06) und der Kompensator (07) eine gleiche bewegte Masse aufweisen.

**14.** Antriebseinheit (01) nach einem der Ansprüche 1 bis 13, weiter umfassend ein längliches Gehäuse, das von einem Benutzer gegriffen werden kann und das Versorgungsanschlüsse aufweist, vorzugsweise für Druckluft und Fluid.

**Revendications**

**1.** Unité d'entraînement (01) pour l'imagerie diagnostique, comprenant :

un oscillateur (06) qui peut être entraîné par un premier actionneur (08) pour se déplacer le long d'un axe longitudinal afin de générer un mouvement oscillant ;
un compensateur (07) qui peut être entraîné par un deuxième actionneur (09) le long de, ou parallèlement à, l'axe longitudinal pour se déplacer en opposition de phase afin de générer un mouvement compensatoire ;
une unité de guidage qui guide de manière axialement mobile l'oscillateur (06) dans un premier guide (11) et le compensateur (07) dans un deuxième guide (13) ;
une unité de contrôle qui commande les premier et deuxième actionneurs (08, 09) pour entraîner l'oscillateur (06) et le compensateur (07),
dans laquelle l'oscillateur (06) comprend un élément oscillant (24) qui est une source de lumière laser ou un capteur ultrasonique, et
le premier guide (11) est un premier guide d'air (11), et le deuxième guide (13) est un deuxième guide d'air (13).

**2.** Unité d'entraînement (01) selon la revendication 1, comprenant en outre un capteur de position qui fournit un signal de positionnement relatif à la position de l'axe longitudinal, de préférence le capteur de position est un capteur d'inclinaison ou un capteur inertiel, ou une combinaison de tels capteurs.

**3.** Unité d'entraînement (01) selon la revendication 2, dans laquelle l'unité de contrôle commande l'oscillateur et le compensateur (07) en tenant compte de la position de l'axe longitudinal.

**4.** Unité d'entraînement (01) selon l'une des revendications 1 à 3, comprenant en outre un capteur de translation (10) qui détecte le mouvement oscillant et le mouvement compensatoire et fournit un signal de translation à l'unité de contrôle.

**5.** Unité d'entraînement (01) selon l'une des revendications 1 à 4, dans laquelle l'oscillateur (06) comprend un chariot oscillant (12) qui est guidé dans le premier guide (11).

**6.** Unité d'entraînement (01) selon l'une des revendications 1 à 5, dans laquelle l'élément oscillant est attaché à l'extrémité distale du chariot oscillant (12).

7. Unité d'entraînement (01) selon l'une des revendications 2 à 6, dans laquelle le premier guide d'air (11) comprend une alimentation en air comprimé, une zone de support proximale (16), une zone d'étanchéité distale (17), et une ouverture de soufflage disposée entre la zone de support (16) et la zone d'étanchéité (17).

8. Unité d'entraînement (10) selon la revendication 7, dans laquelle l'extrémité distale du chariot oscillant (12) s'étend axialement au-delà de la zone d'étanchéité (17) dans une zone de fluide (21), dans laquelle un tuyau d'alimentation (22) débouche pour alimenter en fluide, la zone de fluide (21) comprenant une ouverture (23) pour la sortie du fluide.

9. Unité d'entraînement (01) selon la revendication 8, dans laquelle le fluide est un fluide de couplage, et la zone de fluide est une zone de couplage.

10. Unité d'entraînement (01) selon la revendication 8 ou 9, dans laquelle l'ouverture (23) est une ouverture de couplage (23) pour la sortie du support ? de couplage et/ou de l'ultrason.

11. Unité d'entraînement (01) selon l'une des revendications 8 à 10, dans laquelle des lèvres d'encadrement (05) sont disposées au niveau de l'ouverture (23) pour guider le fluide.

12. Unité d'entraînement (01) selon l'une des revendications 1 à 11, dans laquelle les premier et deuxième actionneurs (08, 09) sont chacun formés par un entraînement bobine de piston.

13. Unité d'entraînement (01) selon l'une des revendications 1 à 12, dans laquelle l'oscillateur (06) et le compensateur (07) ont une masse déplacée égale.

14. Unité d'entraînement (01) selon l'une des revendications 1 à 13, comprenant en outre un boîtier allongé, qui peut être saisi par un utilisateur, et qui a des ports d'alimentation, de préférence pour l'air comprimé et le fluide.

01

03

04

02

09

08

05

Fig. 1

Fig. 2

Fig. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 102018110274
- DE 102018110273
- EP 0035307 A2
- DE 10019226 B4